# FASCICULE DE BREVET EUROPEEN

(11) **EP 2 523 671 B1**
(45) Date de publication et mention de la délivrance du brevet: **02.05.2018**
(21) Numéro de dépôt: 10807463.4
(22) Date de dépôt: 10.12.2010
(51) Int. Cl.: A61K 35/32, A61P 19/02, A61K 35/33

(54) **COMPOSITION PHARMACEUTIQUE DESTINÉE AU TRAITEMENT DES PATHOLOGIES ORTHOPÉDIQUES**
PHARMAZEUTISCHE ZUSAMMENSETZUNG ZUR BEHANDLUNG VON ORTHOPÄDISCHEN KRANKHEITEN
PHARMACEUTICAL COMPOSITION FOR THE TREATMENT OF ORTHOPEDIC PATHOLOGIES

(30) Priorité: 11.12.2009 FR 0958887
(43) Date de publication de la demande: 21.11.2012
(73) Titulaire: Scarcell Therapeutics, 75014 Paris (FR)
(72) Inventeur: GOGLY, Bruno, 77510 Hondevilliers (FR); LAFONT, Antoine, 75007 Paris (FR); COULOMB, Bernard, 91430 Igny (FR)
(74) Mandataire: Vial, Lionel
(86) Numéro de dépôt international: PCT/FR2010/052670
(87) Numéro de publication internationale: WO 2011/070305

(56) Documents cités:
- WO-A1-93/18794
- WO-A1-03/013592
- WO-A1-2009/121761
- WO-A2-01/82773
- MOORE<B C> B A ET AL: "Induction of collagenase-3 (MMP-13) in rheumatoid arthritis synovial fibroblasts", BIOCHIMICA ET BIOPHYSICA ACTA. MOLECULAR BASIS OF DISEASE, AMSTERDAM, NL, vol. 1502, no. 2, 18 octobre 2000 (2000-10-18), pages 307-318, XP004277012, ISSN: 0925-4439, DOI: DOI:10.1016/S0925-4439(00)00056-9
- BROPHY C M ET AL: "Tissue inhibitor of metalloproteases (TIMP) is matrix associated in aortic tissue: Report of a radioimmunoassay", BIOCHEMICAL AND BIOPHYSICAL RESEARCH COMMUNICATIONS, ACADEMIC PRESS INC. ORLANDO, FL, US, vol. 167, no. 3, 30 mars 1990 (1990-03-30) , pages 898-903, XP024769965, ISSN: 0006-291X, DOI: DOI:10.1016/0006-291X(90)90608-P [extrait le 1990-03-30]

## Description

### Domaine de l'invention

La présente invention concerne la prévention et le traitement des pathologies orthopédiques.

### Arrière-plan technique

On considère que la prévalence des pathologies ostéoarticulaires est de l'ordre de 25% de la population des pays industrialisés. Les pathologies orthopédiques, et notamment les pathologies ostéoarticulaires, ciblent les tissus musculo-squelettiques, tels que les os, le cartilage et les membranes synoviales, les ligaments, les tendons et les muscles, qui sont dégradés dans ces pathologies. Les causes de dégradation sont multiples et peuvent impliquer des traumatismes, le vieillissement, une usure mécanique, ou encore une inflammation. A titre d'exemples de pathologies orthopédiques, on peut notamment citer l'ostéoarthrite et la polyarthrite rhumatoïde.

Parmi les différentes stratégies envisagées pour le traitement des pathologies orthopédiques, la thérapie cellulaire apparaît comme un domaine d'avenir.

Ainsi, les cellules souches mésenchymateuses (CSM), des cellules progénitrices non-hématopoïétiques que l'on peut trouver dans différents tissus adultes, tels que la moelle osseuse, le tissu adipeux ou le derme, et qui peuvent donner naissance à certains tissus conjonctifs du squelette, tels que les os et le cartilage, ont été utilisées dans le cadre du traitement de pathologies orthopédiques. En particulier, des tentatives de traitement de maladies arthritiques ont été effectuées à l'aide de CSM (Chen & Tuan (2008) Arthritis Research & Therapy 10:223-234).

Toutefois, il semble que les CSM se greffent de manière relativement inefficace au cartilage articulaire dans le cadre du traitement de l'ostéoarthrite (Chen & Tuan (2008) Arthritis Research & Therapy 10:223-234). Par ailleurs, des résultats contradictoires ont été rapportés pour le traitement de la polyarthrite rhumatoïde, certains auteurs indiquant que les CSM ne permettraient pas d'améliorer l'état des patients (Djouad et al. (2005) Arthritis and Rheumatism 52:1595-1603). Il a été suggéré que ces résultats contradictoires pourraient provenir de l'absence d'une définition claire des CSM ainsi que de leur hétérogénéité.

De ce fait, il y a un besoin pour une alternative aux CSM permettant une prise en charge efficace des pathologies orthopédiques.

Les fibroblastes gingivaux sont des cellules adultes d'origine mésenchymateuse, qui sont capables de migrer, d'adhérer et de proliférer dans les tissus connectifs mous de la gencive. Ils maintiennent ainsi l'intégrité du tissu gingival qui est exposé à de nombreuses agressions, telles que les stress mécaniques, les infections bactériennes, ou les variations de pH et de température. Les fibroblastes gingivaux sont notamment décrits dans Gogly et al., (1997) Clin. Oral Invest. 1:147-152; Gogly et al. (1998) Biochem. Pharmacol. 56:1447-1454; and Ejeil et al. (2003) J. Periodontol. 74:188-195.

Selon les conditions environnementales, les fibroblastes gingivaux sont capables de moduler leur phénotype, et de répondre en proliférant, en migrant et en synthétisant ou en dégradant des composants de la matrice extracellulaire ou des enzymes en lien avec la matrice extracellulaire.

Les fibroblastes gingivaux synthétisent du collagène (par exemple de type I, III, V, VI, VII, XII), des fibres élastiques (de l'oxytalane, de l'élaunine et de l'élastine), des protéoglycanes et des glycosaminoglycanes (par exemple décorine et biglycane), et des glycoprotéines (par exemple la fibronectine et la ténascine). Parallèlement, selon le contexte, les fibroblastes gingivaux synthétisent des enzymes qui sont capables de dégrader les composés macromoléculaires de la matrice (métalloprotéinases matricielles ; MMP), mais également des enzymes inhibant les formes actives des MMP (inhibiteurs de métalloprotéinases ; TIMP). Les fibroblastes gingivaux sont donc des acteurs importants du remodelage de la matrice extracellulaire.

### Résumé de l'invention

La présente invention découle de la mise en évidence inattendue, par les inventeurs, que des fibroblastes gingivaux cultivés avec des chondrocytes, des myocytes, ou des ostéoblastes humains, stimulés par une cytokine pro-inflammatoires, pouvaient inhiber l'activité MMP secrétés par ces cellules, ce qui démontre que les fibroblastes gingivaux sont capables d'inhiber l'activité MMP dans un environnement semblable à celui d'une pathologie orthopédique, notamment inflammatoire.

Ainsi, la présente divulgation concerne une méthode de prévention ou de traitement de pathologies orthopédiques d'un individu, dans laquelle on administre à l'individu une quantité prophylactiquement ou thérapeutiquement effective d'un produit dérivé de fibroblaste gingival.

La présente invention concerne un produit dérivé de fibroblaste gingival sélectionné dans le groupe constitué de cellules entières de fibroblastes gingivaux, d'un milieu conditionné par des fibroblastes gingivaux, d'une culture de fibroblastes gingivaux, et d'un extrait de fibroblaste gingival, obtenu à partir de fibroblastes gingivaux n'ayant pas subi de différenciation en cellules ayant un phénotype ostéogénique, pour son utilisation dans la prévention ou le traitement d'une pathologie orthopédique d'un individu. La présente divulgation concerne également l'utilisation d'un produit dérivé de fibroblaste gingival pour son utilisation dans la préparation d'un médicament destiné à la prévention ou au traitement de pathologies orthopédiques d'un individu.

### Description détaillée de l'invention

Comme on l'entend ici, une pathologie orthopédique selon l'invention est une pathologie ciblant les tissus musculo-squelettiques, à savoir, notamment, les os, le cartilage, les membranes synoviales, les ligaments, les tendons, et les muscles, qui peuvent notamment être dégradés. Ainsi, la pathologie orthopédique selon l'invention est de préférence sélectionnée dans le groupe constitué d'une pathologie orthopédique ostéoarticulaire, d'une pathologie orthopédique musculaire, d'une pathologie orthopédique ligamentaire et d'une pathologie orthopédique tendineuse.

La pathologie orthopédique selon l'invention peut notamment provenir de traumatismes, d'un vieillissement, d'une usure mécanique, ou d'une inflammation d'un tissu musculo-squelettique tel que défini ci-dessus.

En particulier, la pathologie orthopédique selon l'invention est une pathologie osteoarticulaire, plus particulièrement articulaire, notamment inflammatoire. De manière particulièrement préférée, la pathologie orthopédique selon l'invention est la polyarthrite rhumatoïde ou l'ostéoarthrite.

De préférence, l'individu selon l'invention est un mammifère, plus préférablement il s'agit d'un humain.

Dans un mode de réalisation spécifique, les fibroblastes gingivaux selon la divulgation comprennent au moins 75, 80, 90, 95, ou 100% de fibroblastes gingivaux en tant que tels, c'est-à-dire des fibroblastes gingivaux n'ayant subit aucune différenciation, notamment en cellules ayant un phénotype ostéogénique.

Les fibroblastes gingivaux peuvent en outre comprendre des cellules progénitrices, de préférence moins de 25, 20, 15, ou 5 %.

Dans un autre mode de réalisation spécifique, les fibroblastes gingivaux peuvent par exemple être ceux décrits dans Fournier BP et al. (2010) Tissue Eng Part A. 16(9):2891-9.

Les procédures pour prélever, cultiver, et conserver les fibroblastes gingivaux sont bien connues de l'homme du métier et sont notamment décrites dans Naveau et al. (2006) J. Periodontol. 77:238-47 et dans Gogly et al. (2007) Arterioscler. Thromb. Vasc. Biol. 27:1984-90.

Avantageusement, les fibroblastes gingivaux sont aisément prélevables et cultivables. En outre, les fibroblastes gingivaux présentent une vitesse de croissance importante.

De préférence, les fibroblastes gingivaux utilisés selon l'invention sont autologues, c'est-à-dire qu'ils sont prélevés chez l'individu à qui le produit dérivé de fibroblaste doit être administré. Avantageusement, les fibroblastes gingivaux sont une source quasi illimitée de cellules autologues à administrer. Cependant, les fibroblastes gingivaux peuvent également être allogènes, c'est à dire obtenus à partir d'un autre individu de la même espèce, ou encore hétérologues, c'est-à-dire obtenus à partir d'un individu d'une autre espèce.

Comme on l'entend ici, l'expression "produit dérivé de fibroblaste gingival" se réfère à n'importe quel produit susceptible d'être obtenu à partir de fibroblastes gingivaux en eux-mêmes ou qui contient des sécrétions de fibroblastes gingivaux.

Le produit dérivé de fibroblaste gingival selon l'invention est sélectionné dans le groupe constitué de cellules entières de fibroblastes gingivaux, notamment vivantes, d'une culture de fibroblastes gingivaux, d'un extrait de fibroblaste gingival, et d'un milieu conditionné par des fibroblastes gingivaux.

L'extrait de fibroblaste gingival selon l'invention peut être obtenu par n'importe quelle méthode de fragmentation cellulaire connue de l'homme du métier. En particulier, l'extrait de fibroblaste gingival selon l'invention peut être un extrait membranaire, un extrait cytoplasmique ou un extrait nucléaire.

Le milieu conditionné par des fibroblastes gingivaux selon l'invention se réfère à n'importe quel milieu, tel qu'un milieu liquide de culture de cellule (par exemple le milieu « Dulbecco's Modified Eagle Medium », ou un milieu de culture sans sérum), qui a été mis en contact avec des fibroblastes gingivaux, notamment pour une durée suffisante pour que les fibroblastes gingivaux aient pu sécréter des composés dans le milieu.

L'administration à un individu tel que défini ci-dessus d'un produit dérivé de fibroblaste gingival selon l'invention, de préférence à proximité ou au niveau d'un site corporel à traiter, peut être effectuée par n'importe quelle méthode connue de l'homme du métier. On préfèrera toutefois, que le produit dérivé de fibroblaste soit administré par injection à un site de défaut orthopédique. Comme on l'entend ici, un site de défaut orthopédique se réfère à toute zone pathologique d'un tissu musculo-squelettique tel que défini ci-dessus.

De préférence, la méthode de prévention ou de traitement d'un individu selon la divulgation comprend ou consiste en les étapes suivantes :
- prélever des fibroblastes gingivaux d'un individu ;
- cultiver les fibroblastes gingivaux;
- obtenir un produit dérivé de fibroblaste gingival tel que défini ci-dessus à partir des fibroblastes gingivaux cultivés ;
- administrer le produit dérivé de fibroblaste gingival à l'individu.

Lorsque le produit dérivé de fibroblaste gingival est constitué ou comprend des cellules entières, ces cellules peuvent être administrées dans le cadre d'une thérapie cellulaire.

### Description des Figures

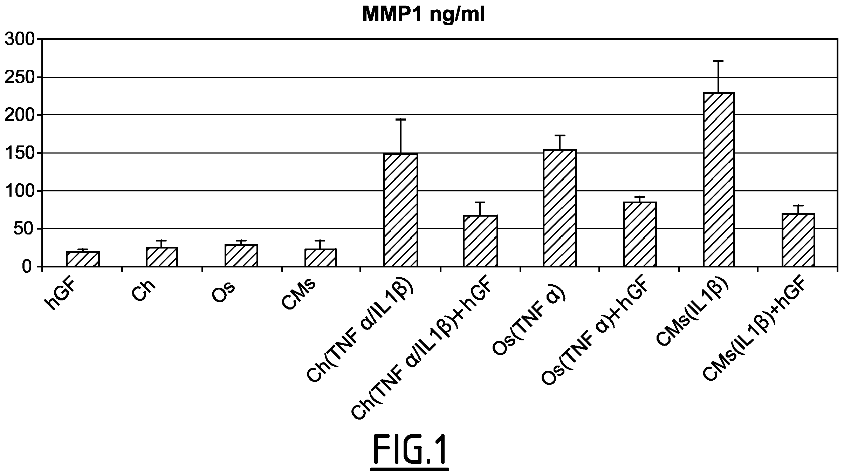

**Figure 1** **:** Quantification par ELISA de MMP1 (en ng/ml) dans les fibroblastes gingivaux (hGF), les chondroblastes (Ch), les ostéoblastes (Os), les cellules musculaires striées (CMs), les chondroblastes stimulés par le TNFα (10 ng/ml) + IL1β (5 ng/ml) seuls (Ch(TNFα/IL1β)) ou en coculture avec les hGF (Ch(TNFα/IL1β)+hGF), les ostéoblastes stimulés par le TNFα (10 ng/ml) seuls (Os(TNFα)) ou en coculture avec les hGF (Os(TNFα)+hGF) et les cellules musculaires striées stimulées par l'IL1β (5 ng/ml) seules (CMs(IL1β)) ou en coculture avec les hGF (CMs(IL1β)+hGF).

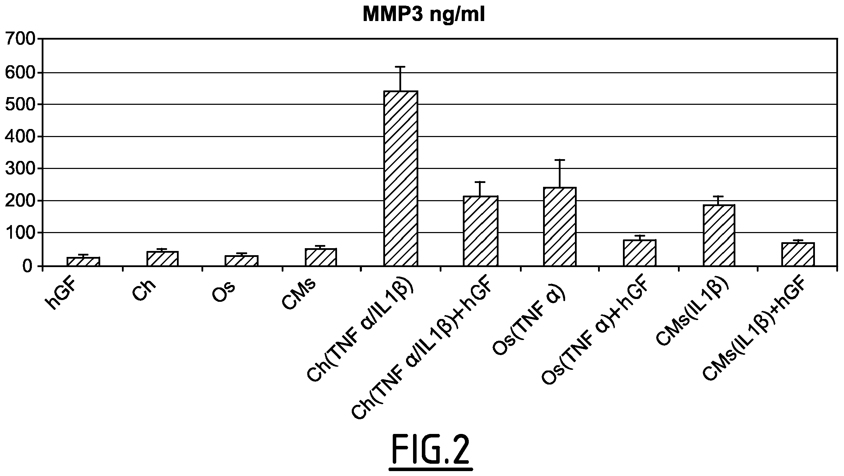

**Figure 2** **:** Quantification par ELISA de MMP3 (en ng/ml) dans les fibroblastes gingivaux (hGF), les chondroblastes (Ch), les ostéoblastes (Os), les cellules musculaires striées (CMs), les chondroblastes stimulés par le TNFα (10 ng/ml) + IL1β (5 ng/ml) seuls (Ch(TNFα/IL1β)) ou en coculture avec les hGF (Ch(TNFα/IL1β)+hGF), les ostéoblastes stimulés par le TNFα (10 ng/ml) seuls (Os(TNFα)) ou en coculture avec les hGF (Os(TNFα)+hGF) et les cellules musculaires striées stimulées par l'IL1β (5 ng/ml) seules (CMs(IL1β)) ou en coculture avec les hGF (CMs(IL1β)+hGF).

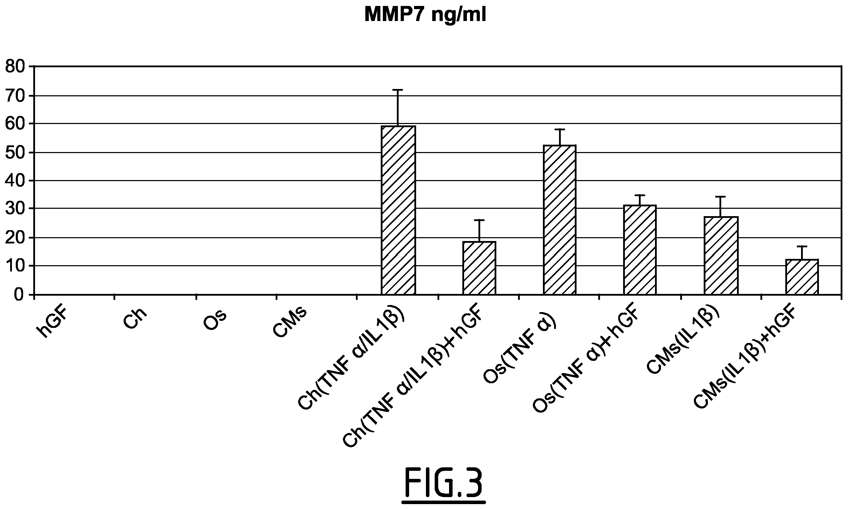

**Figure 3** **:** Quantification par ELISA de MMP7 (en ng/ml) dans les fibroblastes gingivaux (hGF), les chondroblastes (Ch), les ostéoblastes (Os), les cellules musculaires striées (CMs), les chondroblastes stimulés par le TNFα (10 ng/ml) + IL1β (5 ng/ml) seuls (Ch(TNFα/IL1β)) ou en coculture avec les hGF (Ch(TNFα/IL1β)+hGF), les ostéoblastes stimulés par le TNFα (10 ng/ml) seuls (Os(TNFα)) ou en coculture avec les hGF (Os(TNFα)+hGF) et les cellules musculaires striées stimulées par l'IL1β (5 ng/ml) seules (CMs(IL1β)) ou en coculture avec les hGF (CMs(IL1β)+hGF).

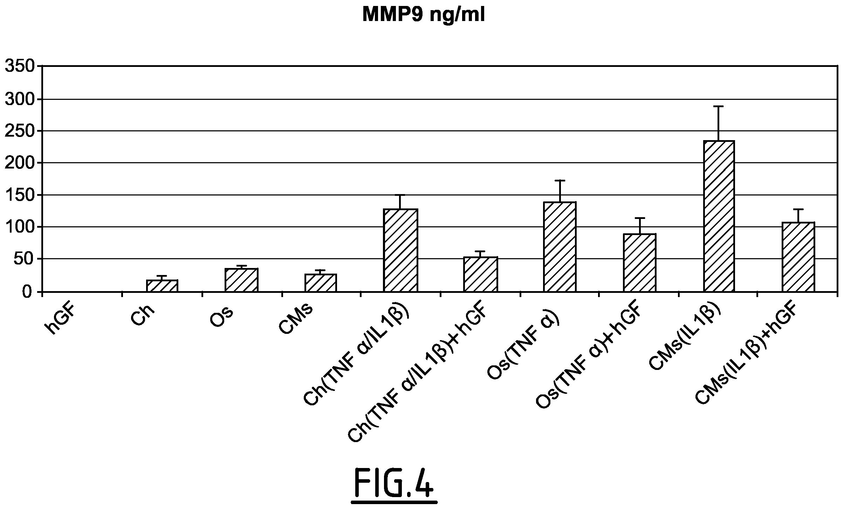

**Figure 4** **:** Quantification par ELISA de MMP9 (en ng/ml) dans les fibroblastes gingivaux (hGF), les chondroblastes (Ch), les ostéoblastes (Os), les cellules musculaires striées (CMs), les chondroblastes stimulés par le TNFα (10 ng/ml) + IL1β (5 ng/ml) seuls (Ch(TNFα/IL1β)) ou en coculture avec les hGF (Ch(TNFα/IL1β)+hGF), les ostéoblastes stimulés par le TNFα (10 ng/ml) seuls (Os(TNFα)) ou en coculture avec les hGF (Os(TNFα)+hGF) et les cellules musculaires striées stimulées par l'IL1β (5 ng/ml) seules (CMs(IL1β)) ou en coculture avec les hGF (CMs(IL1β)+hGF).

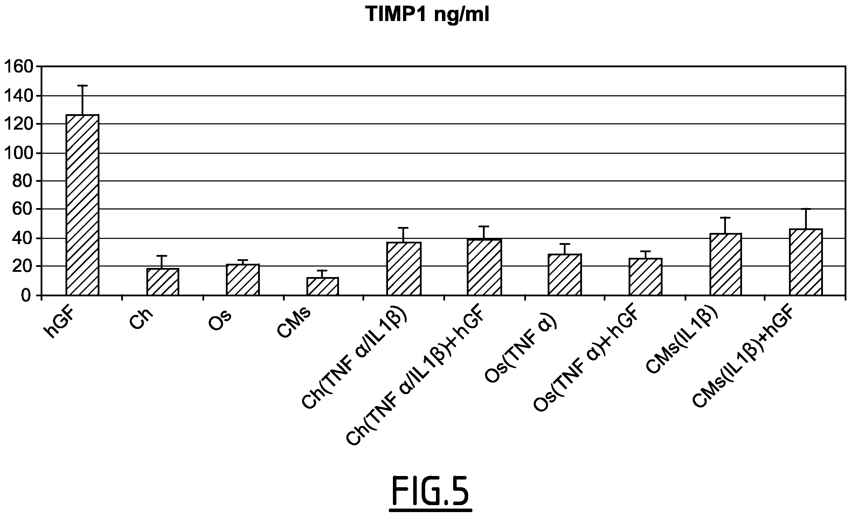

Figure 5 : Quantification par ELISA de TIMP1 (en ng/ml) dans les fibroblastes gingivaux (hGF), les chondroblastes (Ch), les ostéoblastes (Os), les cellules musculaires striées (CMs), les chondroblastes stimulés par le TNFα (10 ng/ml) + IL1β (5 ng/ml) seuls (Ch(TNFα/IL1β)) ou en coculture avec les hGF (Ch(TNFα/IL1β)+hGF), les ostéoblastes stimulés par le TNFα (10 ng/ml) seuls (Os(TNFα)) ou en coculture avec les hGF (Os(TNFα)+hGF) et les cellules musculaires striées stimulées par l'IL1β (5 ng/ml) seules (CMs(IL1β)) ou en coculture avec les hGF (CMs(IL1β)+hGF).

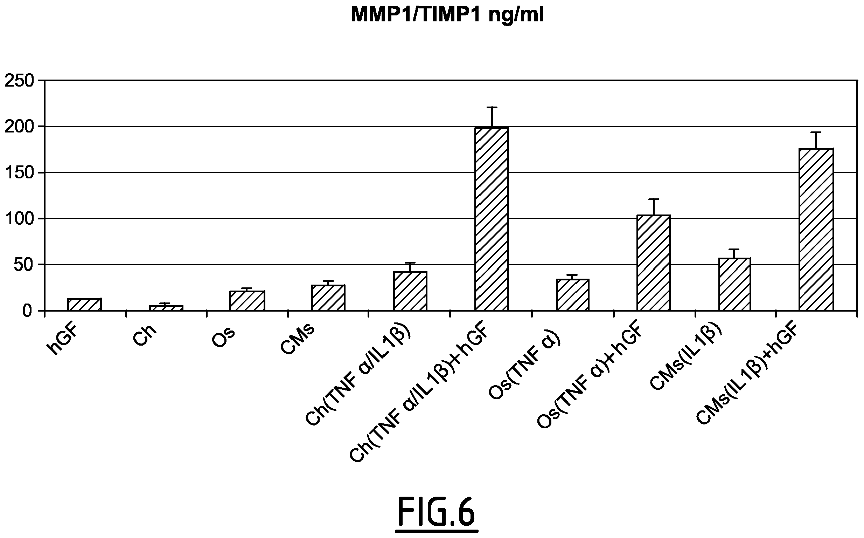

**Figure 6****:** Quantification par ELISA du complexe MMP1/TIMP1 (en ng/ml) dans les fibroblastes gingivaux (hGF), les chondroblastes (Ch), les ostéoblastes (Os), les cellules musculaires striées (CMs), les chondroblastes stimulés par le TNFα (10 ng/ml) + IL1β (5 ng/ml) seuls (Ch(TNFα/IL1β)) ou en coculture avec les hGF (Ch(TNFα/IL1β)+hGF), les ostéoblastes stimulés par le TNFα (10 ng/ml) seuls (Os(TNFα)) ou en coculture avec les hGF (Os(TNFα)+hGF) et les cellules musculaires striées stimulées par l'IL1β (5 ng/ml) seules (CMs(IL1β)) ou en coculture avec les hGF (CMs(IL1β)+hGF).

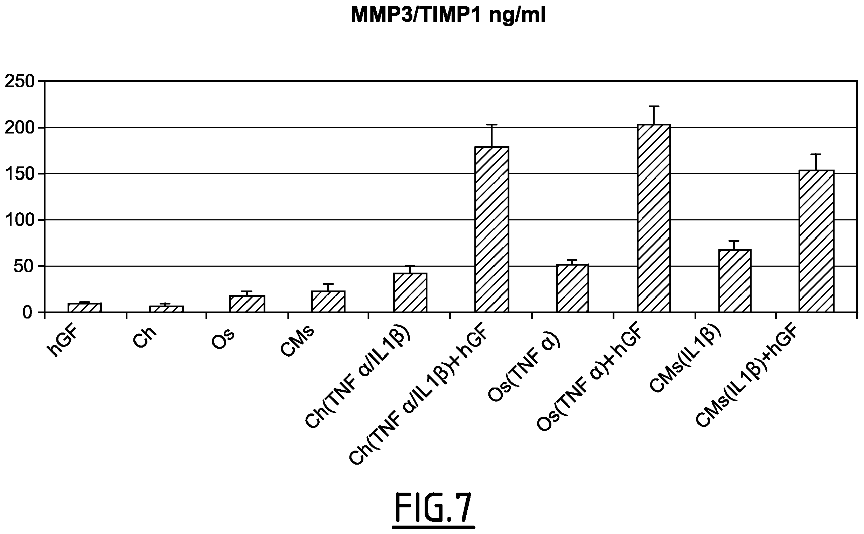

**Figure 7****:** Quantification par ELISA du complexe MMP3/TIMP1 (en ng/ml) dans les fibroblastes gingivaux (hGF), les chondroblastes (Ch), les ostéoblastes (Os), les cellules musculaires striées (CMs), les chondroblastes stimulés par le TNFα (10 ng/ml) + IL1β (5 ng/ml) seuls (Ch(TNFα/IL1β)) ou en coculture avec les hGF (Ch(TNFα/IL1β)+hGF), les ostéoblastes stimulés par le TNFα (10 ng/ml) seuls (Os(TNFα)) ou en coculture avec les hGF (Os(TNFα)+hGF) et les cellules musculaires striées stimulées par l'IL1β (5 ng/ml) seules (CMs(IL1β)) ou en coculture avec les hGF (CMs(IL1β)+hGF).

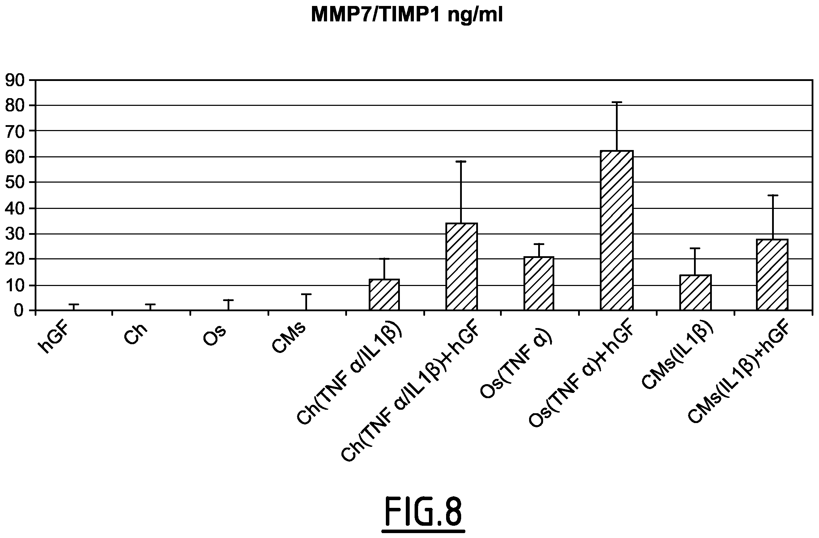

**Figure 8****:** Quantification par ELISA du complexe MMP7/TIMP1 (en ng/ml) dans les fibroblastes gingivaux (hGF), les chondroblastes (Ch), les ostéoblastes (Os), les cellules musculaires striées (CMs), les chondroblastes stimulés par le TNFα (10 ng/ml) + IL1β (5 ng/ml) seuls (Ch(TNFα/IL1β)) ou en coculture avec les hGF (Ch(TNFα/IL1β)+hGF), les ostéoblastes stimulés par le TNFα (10 ng/ml) seuls (Os(TNFα)) ou en coculture avec les hGF (Os(TNFα)+hGF) et les cellules musculaires striées stimulées par l'IL1β (5 ng/ml) seules (CMs(IL1β)) ou en coculture avec les hGF (CMs(IL1β)+hGF).

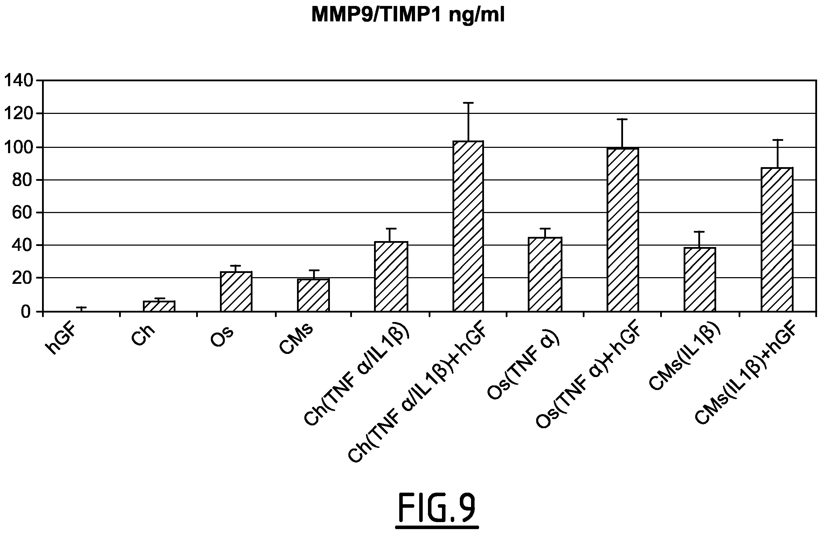

**Figure 9** **:** Quantification par ELISA du complexe MMP9/TIMP1 (en ng/ml) dans les fibroblastes gingivaux (hGF), les chondroblastes (Ch), les ostéoblastes (Os), les cellules musculaires striées (CMs), les chondroblastes stimulés par le TNFα (10 ng/ml) + IL1β (5 ng/ml) seuls (Ch(TNFα/IL1β)) ou en coculture avec les hGF (Ch(TNFa/ILip)+hGF), les ostéoblastes stimulés par le TNFα (10 ng/ml) seuls (Os(TNFα)) ou en coculture avec les hGF (Os(TNFα)+hGF) et les cellules musculaires striées stimulées par l'IL1β (5 ng/ml) seules (CMs(IL1β)) ou en coculture avec les hGF (CMs(IL1β)+hGF).

### EXEMPLE

Cet exemple vise à déterminer si le fibroblaste gingival humain inhibe l'activité de 4 MMP (MMP1, MMP3, MMP7, MMP9) surexprimées par trois cellules clé du remodelage ostéoarticulaire et des pathologies orthopédiques, notamment ostéoarticulaires et musculaires, à savoir : les cellules cartilagineuses (chondroblastes), les ostéoblastes, et les cellules musculaires (cellules musculaires striées).

### Matériel et méthodes

Des cellules clés du remodelage ostéoarticulaire ont été utilisées et mise en culture dans des conditions inflammatoires *in vitro* :
- Chondrocytes humains (c-12710 Promocell)
- Ostéoblaste humains (c-12760 Promocell)
- Cellules Musculaires Striées humaines (c-12580 Promocell)

Ces cellules ont été cultivées dans des milieux spécifiques (Promocell) pour chondroblastes, ostéoblastes et cellules musculaires striées. Les cellules ont été cultivées dans la partie inférieure de *transwell* (Greiner bio-one, réf : 657 641). A confluence les cellules ont été stimulées par une cytokine pro-inflammatoire : TNFα (10 ng/ml) pour les ostéoblastes, IL1β (5 ng/ml) pour les cellules musculaires striées ou l'association de TNFα (10 ng/ml) + IL1β (5 ng/ml) pour les chondroblastes pendant 24h (Pretzel et al. (2009) Arthritis, Res. Ther. 11:R25 ; Moran et al (2009) Arthritis Res. Ther. 11:R113), pour simuler un environnement inflammatoire et permettre l'expression des MMP 1, 3, 7 et 9 comme dans les tissus pathologiques.

Après cette stimulation, les cellules ont ensuite été mises en cocultures dans un milieu DMEM (Dulbecco's Modified Eagle Medium) avec des fibroblastes gingivaux à confluence (partie supérieure du *transwell,* ou ont été cultivées seules (témoin) pendant 24h. Les surnageants de culture ont été analysés au bout de 24h par ELISA pour quantifier l'effet anti-inflammatoire induit par les fibroblastes gingivaux humains (hGF) sur les chondroblastes (Ch), ostéoblastes (Os) et cellules musculaires striées (CMs). Les quantités de MMPs, de TIMP1 (inhibiteur tissulaire de toutes ces MMPs) ainsi que des complexes MMP/TIMP1 ont été quantifiées par ELISA (R&D).

### Résultats

La stimulation des ostéoblastes, des cellules musculaires striées et des chondroblastes par les cytokines a entrainé une augmentation de la sécrétion de toutes les MMPs (Figures 1 à 4). La stimulation de ces trois types cellulaires par les cytokines a donc permis de simuler un environnement inflammatoire comme dans les tissus pathologiques.

En coculture avec les hGF, il a été observé que les concentrations de MMP1, 3, 7, 9 étaient inférieures à celles des cellules cultivées seules après stimulation, et ce pour les trois types cellulaires (ostéoblastes, chondroblastes et cellules musculaires striées) (Figures 1 à 4).

La quantification de TIMP1 dans les hGF a également permis de montrer que TIMP1 est fortement surexprimé dans les hGF (Figure 5).

Il a été observé que les concentrations des complexes TIMP1/MMP1, TIMP1/MMP3, TIMP1/MMP7 et TIMP1/MMP9 étaient plus élevées dans les cocultures avec les hGF que dans les surnageants de culture de cellules témoins. Ces résultats ont donc permis de montrer que le fibroblaste gingival, en surexprimant le TIMP1, inhibe l'activité des MMP 1, 3, 7 et 9 sécrétées par les chondroblastes, ostéoblastes et les cellules musculaires striées stimulées par des cytokines pro-inflammatoires.

Ces résultats démontrent donc que les fibroblastes gingivaux sont capables d'inhiber l'activité des MMPs dans un environnement semblable à celui d'une pathologie orthopédique, et qu'ils conviennent donc au traitement d'une telle pathologie.

## Revendications

1. Produit dérivé de fibroblaste gingival pour son utilisation dans la prévention ou le traitement d'une pathologie orthopédique d'un individu, dans lequel le produit dérivé de fibroblaste gingival est sélectionné dans le groupe constitué de cellules entières de fibroblastes gingivaux, d'un milieu conditionné par des fibroblastes gingivaux, d'une culture de fibroblastes gingivaux, et d'un extrait de fibroblaste gingival, et dans lequel le produit dérivé de fibroblaste gingival est obtenu à partir de fibroblastes gingivaux n'ayant pas subi de différenciation en cellules ayant un phénotype ostéogénique.

2. Produit dérivé de fibroblaste gingival pour son utilisation selon la revendication 1, dans lequel la pathologie orthopédique est sélectionnée dans le groupe constitué d'une pathologie orthopédique ostéoarticulaire, d'une pathologie orthopédique musculaire, d'une pathologie orthopédique ligamentaire et d'une pathologie orthopédique tendineuse.

3. Produit dérivé de fibroblaste gingival pour son utilisation selon la revendication 1 ou 2, dans lequel la pathologie orthopédique est une pathologie ostéoarticulaire.

4. Produit dérivé de fibroblaste gingival pour son utilisation selon l'une des revendications 1 à 3, dans lequel la pathologie orthopédique est une pathologie ostéoarticulaire inflammatoire.

5. Produit dérivé de fibroblaste gingival pour son utilisation selon l'une des revendications 1 à 4, dans lequel la pathologie orthopédique est la polyarthrite rhumatoïde ou l'ostéoarthrite.

6. Produit dérivé de fibroblaste gingival pour son utilisation selon l'une des revendications 1 à 5, dans lequel le produit dérivé de fibroblaste gingival est injecté à l'individu à un site de défaut orthopédique.

7. Produit dérivé de fibroblaste gingival pour son utilisation selon l'une des revendications 1 à 6, dans lequel le produit dérivé de fibroblaste gingival comprend des cellules entières de fibroblastes.

8. Produit dérivé de fibroblaste gingival pour son utilisation selon l'une des revendications 1 à 6, dans lequel le produit dérivé de fibroblaste gingival est un milieu conditionné par des fibroblastes gingivaux.

9. Produit dérivé de fibroblaste gingival pour son utilisation selon l'une des revendications 1 à 6, dans lequel le produit dérivé de fibroblaste gingival est une culture de fibroblastes gingivaux.

10. Produit dérivé de fibroblaste gingival pour son utilisation selon l'une des revendications 1 à 6, dans lequel le produit dérivé de fibroblaste gingival est un extrait de fibroblaste gingival sélectionné dans le groupe constitué d'un extrait membranaire, d'un extrait cytoplasmique et d'un extrait nucléaire.

11. Produit dérivé de fibroblaste gingival pour son utilisation selon l'une des revendications 1 à 10, dans lequel le produit dérivé de fibroblaste gingival est obtenu à partir de fibroblastes gingivaux prélevés chez l'individu.

## Patentansprüche

1. Aus Zahnfleisch-Fibroblasten gewonnenes Produkt zum Gebrauch in der Vorbeugung und Behandlung einer orthopädischen Erkrankung eines Patienten, in dem das aus Zahnfleisch-Fibroblasten gewonnene Produkt aus der Gruppe gewählt ist, bestehend aus ganzen Zahnfleisch-Fibroblasten-Zellen, einem mit Zahnfleisch-Fibroblasten behandelten Medium, einer Zahnfleisch-Fibroblasten-Kultur, und einem Zahnfleisch-Fibroblasten-Auszug und in dem das aus Zahnfleisch-Fibroblasten gewonnene Produkt aus Zahnfleisch-Fibroblasten gewonnen wurde, die keine Differentiation zu Zellen erfahren haben, die einen osteogenen Phänotyp aufweisen.

2. Aus Zahnfleisch-Fibroblasten gewonnenes Produkt zum Gebrauch nach Patentanspruch 1, in dem die orthopädische Erkrankung aus der Gruppe gewählt ist, bestehend aus orthopädischen Gelenkerkrankungen, orthopädischen Muskelerkrankungen, orthopädischen Bändererkrankungen und orthopädischen Sehnenerkrankungen.

3. Aus Zahnfleisch-Fibroblasten gewonnenes Produkt zum Gebrauch nach Patentanspruch 1 oder 2, in dem die orthopädische Erkrankung eine Gelenkerkrankung ist.

4. Aus Zahnfleisch-Fibroblasten gewonnenes Produkt zum Gebrauch nach einem der Patentansprüche 1 bis 3, in dem die orthopädische Erkrankung eine entzündliche Gelenkerkrankung ist.

5. Aus Zahnfleisch-Fibroblasten gewonnenes Produkt zum Gebrauch nach einem der Patentansprüche 1 bis 4, in dem die orthopädische Erkrankung die rheumatoide Polyarthritis oder die Osteoarthritis ist.

6. Aus Zahnfleisch-Fibroblasten gewonnenes Produkt zum Gebrauch nach einem der Patentansprüche 1 bis 5, in dem das aus Zahnfleisch-Fibroblasten gewonnene Produkt dem Patienten in den Sitz eines orthopädischen Defektes injiziert wird.

7. Aus Zahnfleisch-Fibroblasten gewonnenes Produkt zum Gebrauch nach einem der Patentansprüche 1 bis 6, in dem das aus Zahnfleisch-Fibroblasten gewonnene Produkt ganze Zahnfleisch-Fibroblasten-Zellen enthält.

8. Aus Zahnfleisch-Fibroblasten gewonnenes Produkt zum Gebrauch nach einem der Patentansprüche 1 bis 6, in dem das aus Zahnfleisch-Fibroblasten gewonnene Produkt ein mit Zahnfleisch-Fibroblasten behandeltes Medium ist.

9. Aus Zahnfleisch-Fibroblasten gewonnenes Produkt zum Gebrauch nach einem der Patentansprüche 1 bis 6, in dem das aus Zahnfleisch-Fibroblasten gewonnene Produkt eine Zahnfleisch-Fibroblasten-Kultur ist

10. Aus Zahnfleisch-Fibroblasten gewonnenes Produkt zum Gebrauch nach einem der Patentansprüche 1 bis 6, in dem das aus Zahnfleisch-Fibroblasten gewonnene Produkt ein Zahnfleisch-Fibroblasten-Auszug ist, ausgewählt aus der aus einem Membranauszug, einem Zytoplasmaauszug und einem Kernauszug bestehenden Gruppe.

11. Aus Zahnfleisch-Fibroblasten gewonnenes Produkt zum Gebrauch nach einem der Patentansprüche 1 bis 10, in dem das aus Zahnfleisch-Fibroblasten gewonnene Produkt aus Zahnfleisch-Fibroblasten gewonnen wurde, die dem Patienten entnommen wurden.

## Claims

1. A gingival fibroblast-derived product for use in the prevention or treatment of an orthopaedic pathology in an individual, wherein the gingival fibroblast-derived product is selected from the group consisting of gingival fibroblast whole cells, a gingival fibroblast-conditioned medium, a gingival fibroblast culture, and a gingival fibroblast extract, and wherein the gingival fibroblast-derived product is obtained from gingival fibroblasts which have not undergone a differentiation into cells having an osteogenic phenotype.

2. The gingival fibroblast-derived product for use according to claim 1, wherein the orthopaedic pathology is selected from the group consisting of an osteoarticular orthopaedic pathology, a muscular orthopaedic pathology, a ligament orthopaedic pathology and a tendinous orthopaedic pathology.

3. The gingival fibroblast-derived product for use according to claim 1 or 2, wherein the orthopaedic pathology is an osteoarticular pathology.

4. The gingival fibroblast-derived product for use according to any one of claims 1 to 3, wherein the orthopaedic pathology is an inflammatory osteoarticular pathology.

5. The gingival fibroblast-derived product for use according to any one of claims 1 to 4, wherein the orthopaedic pathology is rheumatoid arthritis or osteoarthritis.

6. The gingival fibroblast-derived product for use according to any one of claims 1 to 5, wherein the gingival fibroblast-derived product is injected to the individual at a site of orthopaedic defect.

7. The gingival fibroblast-derived product for use according to any one of claims 1 to 6, wherein the gingival fibroblast-derived product comprises gingival fibroblast whole cells.

8. The gingival fibroblast-derived product for use according to any one of claims 1 to 6, wherein the gingival fibroblast-derived product is a gingival fibroblast-conditioned medium.

9. The gingival fibroblast-derived product for use according to any one of claims 1 to 6, wherein the gingival fibroblast-derived product is a gingival fibroblast culture.

10. The gingival fibroblast-derived product for use according to any one of claims 1 to 6, wherein the gingival fibroblast-derived product is a gingival fibroblast extract selected from the group consisting of a membrane extract, a cytoplasmic extract and a nuclear extract.

11. The gingival fibroblast-derived product for use according to any one of claims 1 to 10, wherein the gingival fibroblast-derived product is obtained from gingival fibroblasts taken from the individual.
